(19) Europäisches Patentamt
European Patent Office
Office européen des brevets

(11) Publication number: 0 001 682
A2

# (12) EUROPEAN PATENT APPLICATION

(21) Application number: 78300414.6

(22) Date of filing: 25.09.78

(51) Int. Cl.[2]: A 01 N 9/22
C 07 D 409/06

(30) Priority: 17.10.77 GB 43064 77

(43) Date of publication of application:
02.05.79 Bulletin 79/9

(84) Designated contracting states:
BE CH DE FR GB NL SE

(71) Applicant: IMPERIAL CHEMICAL INDUSTRIES LIMITED
Imperial Chemical House Millbank
London SW1P 3JF(GB)

(72) Inventor: Sugavanam, Balasubramanyan
11 Kiln Ride
Wokingham Berkshire(GB)

(72) Inventor: Shephard, Margaret Claire
14 Lambourne Drive
Maidenhead Berkshire(GB)

(74) Representative: Fawcett, Richard Fennelly et al,
Imperial Chemical Industries Limited Legal Department:
Patents Thames House North Millbank
London SW1P 4QG(GB)

(54) **Heterocyclic compounds and their use as pesticides.**

(57) Fungicidal triazoles of the formula:-

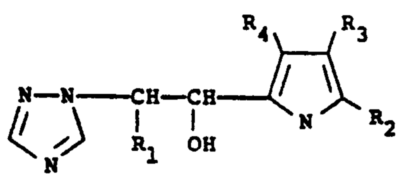

wherein $R_1$ is alkyl, alkenyl, alkynyl or aralkyl, and $R_2$, $R_3$ and $R_4$ are H, halogen, nitro or alkyl, and their ethers, esters, salts and metal complexes.

EP 0 001 682 A2

## Heterocyclic Compounds and their use as Pesticides

This invention relates to heterocyclic compounds which are 1,2,4-triazole compounds, to a process for preparing them, to compositions comprising them and to a method of combating fungal diseases in plants using them.

German Offenlegungsschrift No P 26 45 152 discloses certain triazolylmethyl thienyl ketones and their use as plant fungicides. We have now found that the corresponding hydroxy compounds have fungicidal activity.

The invention therefore provides a compound of general formula (I):

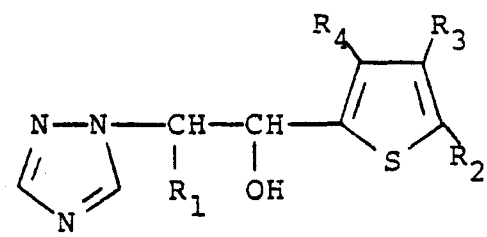

wherein $R_1$ is alkyl, alkenyl, alkynyl or optionally substituted aralkyl and each of $R_2$, $R_3$ and $R_4$, which may be the same or different, is hydrogen, halogen, nitro or alkyl, or an ester, an ether, an acid addition salt or a metal complex thereof.

The compounds of the invention contain chiral centres. The compounds are generally obtained in the form of racemic mixtures. However these or other

mixtures can be separated into the individual isomers by methods known in the art e.g. chromatography. In many cases, the compounds can be prepared stereospecifically in the form of a single diastereoisomer.

The alkyl, alkenyl and alkynyl groups, which can be straight or branched chain, preferably have up to 5 carbon atoms; examples are methyl, ethyl, propyl (n- or i-propyl), butyl (n-, sec-, i- or t-butyl), allyl and propargyl.

The aralkyl group suitably contains 7 to 12 carbon atoms. The aralkyl (e.g. benzyl) group can be substituted in its alkyl (e.g. $CH_2$) and/or aryl (e.g. phenyl) moieties. Suitable substituents on its aryl (e.g. phenyl) moiety are halogen, $C_{1-4}$ alkyl [e.g. methyl, ethyl, propyl (n- or i-propyl) and butyl (n-, i- or t-butyl)], halo-($C_{1-4}$ alkyl), phenyl, halophenyl (e.g. chlorophenyl), cycloalkyl, nitro, cyano, $C_{1-4}$ alkoxy (e.g. methoxy or ethoxy), ($C_{1-4}$ alkylene)dioxy (e.g. methylenedioxy), ($C_{1-4}$ alkoxy) ($C_{1-4}$ alkyl) [e.g. 2-methoxy- or ethoxy-ethyl], mercapto, ($C_{1-4}$ alkyl) thio [e.g. methyl- or ethyl-thio], ($C_{1-4}$ alkyl) sulphonyl [e.g. methyl- or ethyl-sulphonyl], ($C_{1-4}$ halo-alkyl)sulphonyl [e.g. trifluoromethylsulphonyl], phenyl-sulphonyl, unsubstituted or mono- or di-($C_{1-4}$ alkyl) substituted sulphamoyl or carbamoyl, carboxy, ($C_{1-4}$ alkoxy)-carbonyl [e.g. methoxy- or ethoxy-carbonyl], unsubstituted or mono- or di- ($C_{1-4}$ alkyl) substituted amino, ($C_{1-6}$ alkanoyl)amino, N-($C_{1-4}$ alkyl)-substituted ($C_{1-6}$ alkanoyl)-amino, formylamino, N-($C_{1-4}$ alkyl)-substituted formylamino, phenylethyl, phenoxy or benzyloxy. A suitable alkanoyl is acetyl or propionyl. The aralkyl group can have more than one ring substituent; examples of polysubstituted groups are those substituted with up to the maximum possible number (especially 1, 2 or 3) of for example halogen (particularly chlorine) atoms and/or nitro, methyl or methoxy groups. Suitable

substituents on the alkyl moiety of the aralkyl (e.g. benzyl) group are halogen, $C_{1-4}$ alkyl (e.g. methyl), phenyl or benzyl, both latter groups being optionally substituted as indicated above for aryl, cyano, ($C_{1-4}$ alkoxy)carbonyl [e.g. methoxy-or ethoxy-carbonyl] or trihalomethyl (e.g. trifluoro-methyl).

Examples of suitable aralkyl groups are benzyl itself, α-methylbenzyl, α-methylchlorobenzyl (e.g. α-methyl-p-chlorobenzyl), α-methyldichlorobenzyl (e.g. α-methyl-2,4-dichlorobenzyl), α-methylfluorobenzyl [e.g. α-methyl-p-fluorobenzyl], chlorobenzyl (for example o-, m- or p-chloro-benzyl), dichlorobenzyl (e.g. 3,4-, 2,4- or 2,6-dichlorobenzyl), trichlorobenzyl (e.g. 2,3,6- or 2,4,5-trichlorobenzyl), tetrachlorobenzyl, pentachlorobenzyl, bromobenzyl (e.g. o-, m- or p-bromobenzyl), dibromobenzyl (e.g. 2,4-dibromobenzyl), fluorobenzyl (e.g. o-, m- or p-fluorobenzyl), difluorobenzyl (e.g. 2,4-difluorobenzyl), pentafluorobenzyl, methylbenzyl (e.g. o-, m- or p-methylbenzyl), dimethylbenzyl (e.g. 2,5-dimethylbenzyl), cyanobenzyl (e.g. p-cyanobenzyl), nitro-benzyl (e.g. p-nitrobenzyl), (trifluoromethyl)benzyl [e.g. m-(trifluoromethyl)-benzyl], methoxybenzyl (e.g. o-, m- or p-methoxybenzyl), chloronitrobenzyl (e.g. 3-nitro-4-chlorobenzyl), chlorofluorobenzyl (e.g. 4-chloro-2-fluorobenzyl), fluorobromobenzyl (e.g. 2-fluoro-4-bromo-benzyl), methoxybromobenzyl (e.g. 2-methoxy-5-bromobenzyl), phenylbenzyl (e.g. p-phenylbenzyl), phenylethyl (e.g. 2-phenylethyl) or naphthylmethyl. In this paragraph, all the substituents except the α-methyl substituents are ring substituents.

The halogen can be fluorine, chlorine, bromine or iodine.

Suitable salts are salts with inorganic or organic acids, e.g. hydrochloric, nitric, sulphuric, toluenesulphonic, acetic or oxalic acid. The esters are

suitably alkanoates (e.g. acetates) and the ethers are suitably alkyl (e.g. methyl or ethyl), aryl (e.g. phenyl) or aralkyl (e.g. benzyl) ethers.

The metal complex is suitably one including copper, zinc, manganese or iron. It preferably has the general formula:

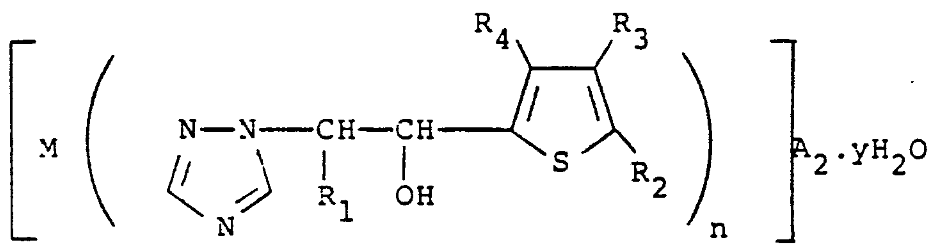

wherein $R_1$ to $R_4$ are as defined above, M is a metal, A is an anion (e.g. a chloride, bromide, iodide, nitrate, sulphate or phosphate anion), n is 2 or 4, and y is O or an integer of 1 to 12.

Specific examples of the compounds are given in Table I.

TABLE I

| NO | $R_1$ | $R_2$ | $R_3$ | $R_4$ | MELTING POINT ($^{\circ}C$) |
|---|---|---|---|---|---|
| 1* | n-Bu | Cl | H | H | Oil |
| 2* | $p-Cl-C_6H_4-CH_2$ | Cl | H | H | $50-80^{\circ}$ |
| 3 | $p-NO_2-C_6H_4-CH_2$ | Cl | H | H | $191-193^{\circ}$ |
| 4 | i-Pr | Cl | H | H | $119-120^{\circ}$ |
| 5 | $C_6H_5CH_2-$ | Cl | H | H | $153-155^{\circ}$ |
| 6 | $2,4-diCl-C_6H_3CH_2-$ | Cl | H | H | $124-126^{\circ}$ |
| 7 | $o-F-C_6H_4-CH_2-$ | Cl | H | H | $124-126^{\circ}$ |
| 8 | $2-F,4-Cl-C_6H_3CH_2-$ | Cl | H | H | $114-116^{\circ}$ |
| 9 | $2,4-diCl-C_6H_3CH_2$ | H | H | H | $110-112^{\circ}$ |
| 10 | $o-F-C_6H_4CH_2$ | H | H | H | $138-139^{\circ}$ |
| 11* | $CH=CCH_2$ | H | H | H | $111-113^{\circ}$ |
| 12* | $p-Cl-C_6H_4CH_2$ | H | H | H | semi-solid |
| 13 | $p-F-C_6H_4CH_2$ | H | H | H | oil |
| 14 | i-Bu | H | H | H | oil |
| 15 | $CH_2=CHCH_2$ | Cl | H | H | $95-97^{\circ}$ |

* Nuclear magnetic resonance studies have shown that each of Compounds 1 and 2 is in the form of a mixture of stereoisomers.

The compounds of general formula (I), or a salt thereof, can be prepared by reducing, preferably at 0 to 100$^{\circ}$C and for 1 to 12 hours, a compound of general formula (II):

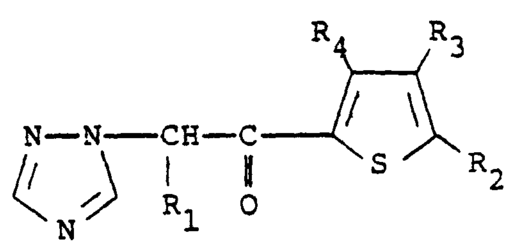

wherein $R_1$ to $R_4$ are as defined above, or a salt thereof. Suitable reducing agents are sodium borohydride, lithium aluminium hydride or aluminium isopropoxide. If desired, catalytic hydrogenation using a suitable metal catalyst can be used.

The reduction can be performed by dissolving the reactants in a solvent such as diethyl ether or tetrahydrofuran (for lithium aluminium hydride reduction) or hydroxylic solvents (for sodium borohydride reduction). The reaction temperature will depend on the reactants and solvent; but generally the reaction mixture is heated under reflux. After the reaction, the product can be isolated by extraction into a convenient solvent after acidification with dilute mineral acid. On removal of the solvent *in vacuo*, the product may be crystallised from a convenient solvent.

The starting compound of general formula (II) may be made by reacting 1,2,4-triazole, or a salt thereof, with a α-haloketone of general formula (III):-

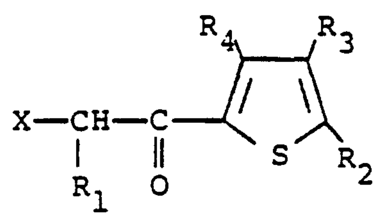

wherein $R_1$ to $R_4$ are as defined above, and X is halogen, preferably bromine or chlorine. This process may be carried out by heating the reactants together in the absence of a solvent or diluent, but preferably a solvent is present. This process is discussed in more detail in German Offenlegungsschrift No. P26 45 152, the disclosure of which document is incorporated herein by reference.

The compounds of general formula (II) or a salt thereof may also be made by alkylating or aralkylating a compound of general formula (IV):-

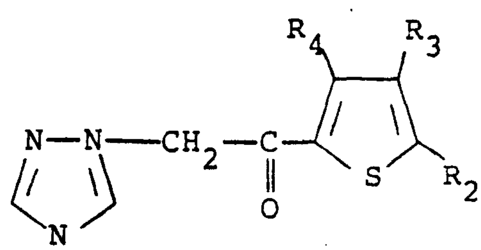

wherein Y and $R_2$ are as defined above. Further details of a reaction related to this reaction can be found in German Offenlegungsschrift No. 2610022, the disclosure of which document is incorporated herein by reference.

The salts, metal complexes, ethers and esters of the compounds of general formula (I) can be prepared from the latter in known manner. For example, the complexes can be made by reacting the uncomplexed compound with a metal salt in a suitable solvent.

The compounds are active fungicides, particularly against the diseases:-

_Piricularia oryzae_ on rice
_Puccinia recondita_, _Puccinia striiformis_ and other rusts on wheat, _Puccinia hordei_, _Puccinia striiformis_ and other rusts on barley, and rusts on other hosts e.g. coffee, apples, vegetables and ornamental plants
_Plasmopara viticola_ on vines
_Erysiphe graminis_ (powdery mildew) on barley and wheat and other powdery mildews on various hosts such as _Sphaerotheca fuliginea_ on cucurbits (e.g. cucumber), _Podosphaera leucotricha_ on apples and _Uncinula necator_ on vines
_Helminthosporium_ spp. on cereals
_Cercospora arachidicola_ on peanuts and other _Cercospora_ species on for example sugar beet, bananas and soya beans
_Botrytis cinerea_ (grey mould) on tomatoes, strawberries, vines and other hosts
_Phytophthora infestans_ (late blight) on tomatoes
_Venturia inaequalis_ (scab) on apples

Some of the compounds have also shown a broad range of activities against fungi *in vitro*. They have activity against various post-harvest diseases on fruit (e.g. *Penicillium digatatum* and *italicum* on oranges and *Gloeosporium musarum* on bananas). Further some of the compounds are active as seed dressings against: *Fusarium* spp., *Septoria* spp., *Tilletia* spp. (i.e. bunt, a seed borne disease of wheat), *Ustilago* spp., *Helminthosporium* spp. on çereals, *Rhizoctonia solani* on cotton and *Corticium sasakii* on rice.

The compounds of general formula (I) have in general higher activity against powdery mildews on wheat and barley than the corresponding ketones of German Offenlegungsschrift No P26 45 152 and some of them have higher activity against rust on wheat. Further, they have in general lower phytotoxicity than the corresponding ketones.

The compounds also have plant growth regulating activities, e.g. a stunting or dwarfing effect on the vegetative growth of woody and herbaceous mono- and dicotyledonous plants. They also have anti-bacterial and anti-viral activities as well as herbicidal activities.

The compounds may be used as such for fungicidal purposes but are more conveniently formulated into compositions for such usage. The invention thus provides also a fungicidal composition comprising a compound of general formula (I) or a salt, complex, ether or ester thereof as hereinbefore defined, and a carrier or diluent.

The invention also provides a method of combating fungal diseases in a plant, which method comprises applying to the plant, to seed of the plant or to the locus of the plant or seed a compound or salt, complex, ether or ester thereof as hereinbefore defined.

The compounds, salts, complexes, ethers and esters can be applied in a number of ways, for example they

can be applied, formulated or unformulated, directly to the foliage of a plant, to seeds or to other medium in which the plants are growing or are to be planted, or they can be sprayed on, dusted on or applied as a cream or paste formulation, or they can be applied as a vapour. Application can be to any part of the plant, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a microencapsulated form.

By including suitable additives, for example additives for improving the distribution, adhesive power and resistance to rain on treated surfaces, the different compositions can be better adapted for various utilities.

The compounds can be used as mixtures with fertilisers (e.g. nitrogen-, potassium- or phosphorus-containing fertilisers). Compositions comprising only

granules of fertiliser incorporating, for example coated with, the compound, are preferred. Such granules suitably contain up to 25% by weight of the compound. The invention therefore also provides a fertiliser composition comprising the compound of general formula (I) or a salt, metal complex, ether or ester thereof.

The compositions may also be in the form of liquid preparations for use as dips or sprays which are generally aqueous dispersions or emulsions containing the active ingredient in the presence of one or more surfactants e.g. wetting agent(s), dispersing agent(s), emulsifying agent(s) or suspending agent(s). These agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a

concentrate containing a high proportion of the active ingredient(s), the concentrate to be diluted with water before use. These concentrates often should be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient(s). These concentrates suitably contain organic acids (e.g. alkaryl or aryl sulphonic acids such as xylenesulphonic acid or dodecylbenzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having similar or complementary fungicidal or compounds having herbicidal, plant growth regulating or insecticidal activity.

The other fungicidal compound can be for example one which is capable of combating ear diseases of cereals (e.g. wheat) such as <u>Septoria</u>, <u>Gibberella</u> and <u>Helminthosporium</u> spp., seed and soil borne diseases and downy and powdery mildews on grapes and powdery mildew and scab on apple etc. These mixtures of fungicides can have a broader spectrum of activity than the

 

compound of general formula (I) alone; further the other fungicide can have a synergistic effect on the fungicidal activity of the compound of general formula (I). Examples of the other fungicidal compounds are imazalil, benomyl, carbendazim (BCM), thiophanate-methyl, captafol, captan, sulphur, dithiocarbamates, carbathiins, copper oxychloride, triforine, dodemorph, tridemorph, dithianon, pyrazophos, binapacryl, quino-methionate, panoctine, furalaxyl, aluminium trio(ethyl phosphonate), DPX3217, ethirimol, dimethirimol, bupirimate, chlorothalonil and metaxanine.

The compounds of general formula (I) can ben mixed with soil, peat or other rooting media for the protection of plants against seed-borne, soil-borne or foliar fungal diseases.

The plant growth regulating compounds can be growth stimulating substances such as the gibberellins (e.g. $GA_3$, $GA_4$ or $GA_7$), the auxins (e.g. indoleacetic or indolebutyric acid) and the cytokinins (e.g. kinetin, diphenylurea, benzimidazole and benzyladenine).

The compositions can comprise also stabilising agent(s), for example epoxides (e.g. epichlorhydrin).

The following Examples illustrate the invention; the temperatures are given in degrees Centigrade ($^{O}$).

## EXAMPLE 1

<u>1-(5-Chloro-thien-2-yl)-2-(1,2,4-triazol-1-yl)-hexan-1-ol</u> (Compound 1)

A solution of α-1,2,4-triazol-1-yl-α-n-butyl-5-chloro-2-acetylthiophene (1.0 g; prepared according to Example 1 of German Offenlegungsschrift No P26 45 152) in methanol (10 ml) was treated portionwise at room temperature with sodium borohydride (0.13 g). After

the initial vigorous reaction had subsided, the reaction mixture was refluxed for one hour and the solvent was removed *in vacuo*. 1N-hydrochloric acid (10 ml) was added to the residue and the aqueous solution was extracted into chloroform. The organic layer was washed with water, dried ($MgSO_4$) and the solvent was removed to give the title product as an oil; it was in the form of a mixture of diastereoisomers.

EXAMPLE 2

The compounds were tested against a variety of foliar fungal diseases of plants. The technique employed was as follows.

The plants were grown in John Innes Potting Compost (No. 1, or Seed, as appropriate) in 4 cm diameter mini-pots. A layer of fine sand was placed at the bottom of the pot to facilitate uptake of test compound by the roots.

The test compounds were formulated whether by bead-milling with aqueous Dispersol T or as a solution in acetone/ethanol which was diluted to the required concentration immediately before use. For the foliage diseases, 100 p.p.m. a.i. suspensions were sprayed on to the foliage and applied to the roots of the same plant *via* the soil. (Sprays were applied to maximum retention, and root drenches to a final concentration equivalent to approximately 40 ppm a.i./dry soil). Tween 20, to give a final concentration of 0.1%, was added when the sprays were applied to the cereals.

For most of the tests, the test compound was applied to the soil (roots) and to the foliage (by spraying) one or two days before the plant was inoculated with the diseases. An exception was the test on *Erysiphe graminis*, in which the plants were inoculated 24 hours before treatment. After inoculation, the plants were put into an appropriate environment to allow infection to take place and then incubated until the disease was ready for assessment. The period between inoculation and assessment varied from 4 to 14 days according to the disease and environment.

The disease control was recorded by the following grading:

4 = No disease
3 = 0-5%
2 = 6-25%
1 = 26-60%
0 = >60%

The results are shown in Table II.

TABLE II

| COMPOUND NO | PUCCINIA RECONDITA (WHEAT) | ERYSIPHE GRAMINIS (BARLEY) | PIRICULARIA ORYZAE (RICE) | CERCOSPORA ARACHIDICOLA (PEANUT) | PLASMOPARA VITICOLA (VINE) | PHYTOPTHORA INFESTANS (TOMATO) | BOTRYTIS CINEREA (TOMATO) |
|---|---|---|---|---|---|---|---|
| 1 | 3 | 4 | | | 4 | 0 | 3 |
| 2 | 4 | 4 | 2 | | 0 | 1 | 0 |
| 3 | 3 | 4 | 1 | | 0 | 0 | 0 |
| 4 | 3 | 4 | 2 | | 0 | 3 | 0 |
| 5 | 4 | 4 | 0 | | | 0 | 2 |
| 6 | 3 | 4 | 2 | | 0 | 0 | 3 |
| 7 | 4 | 4 | 0 | | 0 | 0 | 3 |
| 8 | 4 | 3 | 0 | 2 | 0 | 0 | 1 |
| 9 | 0 | 4 | 1 | | 0 | 0 | 2 |
| 10 | 4 | 4 | 2 | | 0 | 0 | 1 |
| 11 | 1 | 3 | 2 | | 0 | 0 | 0 |
| 12 | 3 | 4 | 0 | | 1 | | 0 |
| 13 | 4 | 4 | 2 | | 0 | 0 | 0 |
| 14 | 4 | 4 | 3 | 2 | 0 | 0 | 0 |
| 15 | 4 | 4 | 4 | 3 | 0 | 0 | 0 |

0001682

1. A compound of general formula (I):

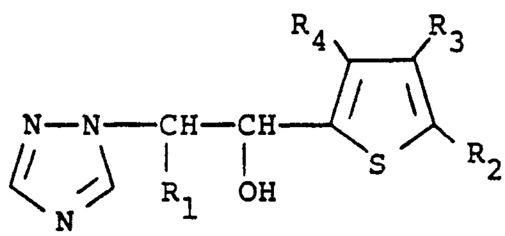

wherein $R_1$ is alkyl, alkenyl, alkynyl or optionally substituted aralkyl and each of $R_2$, $R_3$ and $R_4$, which may be the same or different, is hydrogen, halogen, nitro or alkyl, or an ester, an ether, an acid addition salt or a metal complex thereof.

2. A compound according to Claim 1 wherein $R_1$ is $C_{1-5}$ alkyl or $C_{2-5}$ alkenyl or alkynyl.

3. A compound according to Claim 2 wherein $R_1$ is i-propyl, i-butyl, t-butyl, allyl or propargyl.

4. A compound according to Claim 1 wherein $R_1$ is aralkyl optionally substituted on its aryl moiety with halogen, $C_{1-4}$ alkyl, halo-($C_{1-4}$ alkyl), phenyl, halophenyl, cycloalkyl, nitro, cyano, $C_{1-4}$ alkoxy, ($C_{1-4}$ alkylene)dioxy, ($C_{1-4}$ alkoxy) ($C_{1-4}$ alkyl), mercapto, ($C_{1-4}$ alkyl)thio, ($C_{1-4}$ alkyl)-sulphonyl, ($C_{1-4}$ halo-alkyl)sulphonyl, phenyl-sulphonyl, unsubstituted or mono-or di-($C_{1-4}$ alkyl) substituted sulphamoyl or carbamoyl, carboxy, ($C_{1-4}$ alkoxy)-carbonyl, unsubstituted or mono-or di- ($C_{1-4}$ alkyl) substituted amino, ($C_{1-6}$ alkanoyl)amino, N-($C_{1-4}$ alkyl) substituted ($C_{1-6}$ alkanoyl)-amino, formylamino, N-($C_{1-4}$ alkyl) substituted formylamino, phenylethyl, phenoxy or benzyloxy, and/or on its alkyl moiety with phenyl or benzyl optionally substituted as indicated

above for aryl, or with halogen, $C_{1-4}$ alkyl, cyano, ($C_{14}$ alkoxy)carbonyl or trihalomethyl.

5. A compound according to Claim 4 wherein $R_1$ is benzyl, α-methylbenzyl, α-methylchlorobenzyl, α-methyldichlorobenzyl, α-methylfluorobenzyl, chlorobenzyl, dichlorobenzyl, trichlorobenzyl, tetrachlorobenzyl, pentachlorobenzyl, bromobenzyl, dibromobenzyl, fluorobenzyl, difluorobenzyl, pentafluorobenzyl, methylbenzyl, dimethylbenzyl, cyanobenzyl, nitrobenzyl, (trifluoromethyl)benzyl, methoxybenzyl, chloronitrobenzyl, chlorofluorobenzyl, fluorobromobenzyl, methoxybromobenzyl, phenylbenzyl, phenylethyl or naphthylmethyl, the substituents except the α-methyl substituents being all ring substituents.

6. A compound according to Claim 5 wherein $R_1$ is benzyl, p-chlorobenzyl, 2,4-dichlorobenzyl, o- or p-fluorobenzyl or 2-fluoro-4-chlorobenzyl.

7. A compound according to any one of the preceding Claims wherein $R_2$ is hydrogen or chlorine, and $R_3$ and $R_4$ are hydrogen.

8. A fungicidal composition comprising, as active ingredient, a compound, ester, ether, salt or metal complex according to any one of Claims 1 to 7, and a carrier for the active ingredient.

9. A method of combating fungal diseases in a plant, the method comprising applying to the plant, to seed of the plant or to the locus of the plant or seed, a compound, ester, ether, salt or metal complex according to any one of Claims 1 to 7.